# EUROPÄISCHE PATENTANMELDUNG

(11) **EP 1 767 519 A1**
(43) Veröffentlichungstag der Anmeldung: **28.03.2007**
(21) Anmeldenummer: 06019621.9
(22) Anmeldetag: 20.09.2006
(51) Int. Cl.: C07C 201/06, C07C 205/03

(54) **Hochenergetische Verbindung**

(30) Priorität: 23.09.2005 DE 102005045419
(71) Anmelder: Diehl BGT Defence GmbH & Co.KG, 88662 Überlingen (DE)
(72) Erfinder: Koch Ernst-Christian Dr., 67657 Kaiserslautern (DE)
(74) Vertreter: Diehl Patentabteilung

(57) **Zusammenfassung**

Es wird eine neue hochenergetische Verbindung vorgeschlagen, die in vorteilhafter Weise zum Beispiel als Sprengmittel, Treibmittel, Oxidationsmittel und dergleichen einsetzbar ist, umfassend eine Alkineinheit, bei welcher wenigstens ein Wasserstoffatom durch eine Trinitromethylgruppe substituiert ist.

## Beschreibung

Die vorliegende Erfindung betrifft eine neue hochenergetische Verbindung, die in vorteilhafter Weise zum Beispiel als Sprengmittel, Treibmittel, Oxidationsmittel und dergleichen einsetzbar ist.

Unter den einheitlichen energetischen Materialien zum Einsatz als Spreng- und Treibmittel beanspruchen die Nitrokohlenstoffe der allgemeinen Zusammensetzung Cₙ(NO₂)ₘ besonderes Interesse. Die Nitrokohlenstoffe lassen sich je nach Hybridisierungsgrad des Kohlenstoffs in die Pernitroalkane, Pernitroalkene, Pernitroaromaten und Pernitroacetylene unterteilen. Die Gruppe der homoleptischen Nitrokohlenstoffverbindungen umfasst bis heute Tetranitromethan C(NO₂)₄, Hexanitroethan C₂(NO₂)₆, Tetranitroethylen C₂(NO₂)₄, Hexanitrobenzol C₆(NO₂)₆, Decanitrobiphenyl C₁₂(NO₂)₁₀ sowie das kürzlich synthetisierte Octanitrocuban C₈(NO₂)₈.

Für die linearen Alkane haben Rechnungen gezeigt, dass die starken Van-der-Waals-Kräfte in Molekülen wie Octanitropropan und Decanitrobutan zu einer erheblichen Destabilisierung der C-N - Bindung führen, sodass diese Substanzen sicher nicht stabil sind. Auch zeigt die langsame Zersetzung von Hexanitroethan (nachfolgend kurz als HNE bezeichnet) bereits bei 50°C, dass bei höheren Homologen mit einer noch schnelleren Zersetzung zu rechnen ist.

Der vorliegenden Erfindung liegt die Aufgabe zugrunde, eine hochenergetische und gleichzeitig ausreichend stabile Verbindung bereitzustellen.

Diese Aufgabe wird gelöst durch eine hochenergetische Verbindung mit den Merkmalen des Anspruchs 1. Vorteilhafte Ausgestaltungen und Weiterbildungen der Erfindung sind Gegenstand der abhängigen Ansprüche.

Die neue hochenergetische Verbindung der Erfindung umfasst eine Alkineinheit, bei welcher wenigstens ein Wasserstoffatom durch eine Trinitromethylgruppe substituiert ist. In einer derartigen Verbindung sind die Trinitromethylgruppen durch die Alkineinheit ausreichend weit voneinander getrennt, sodass keine die thermische Zersetzung begünstigende C-N - Bindungsschwächung existiert.

In einer vorteilhaften Weiterbildung ist wenigstens ein weiteres Wasserstoffatom der Alkineinheit durch einen Rest substituiert, der ausgewählt ist aus der Gruppe bestehend aus NO₂, NO, NH₂, N(NO₂)₂ und einem Alkyl.

Die hochenergetische Verbindung ist bevorzugt ein Trinitromethylacetylenderivat der Formel C₃R(NO₂)₃, wobei R ausgewählt ist aus der Gruppe bestehend aus H, NO₂, NO, NH₂, N(NO₂)₂, C(NO₂)₃ und einem Alkyl, und insbesondere Bis(trinitromethyl) acetylen C₄(NO₂)₆ für R = C(NO₂)₃, oder ein Trinitromethylbutadiinderivat der Formel C₅R¹R²R³(NO₂)₃, wobei R¹, R² und R³ jeweils ausgewählt sind aus der Gruppe bestehend aus H, NO₂, NO, NH₂, N(NO₂)₂, C(NO₂)₃ und einem Alkyl, und insbesondere Bis(trinitromethyl)butadiin C₆H₂(NO₂)₆ für R¹ = C(NO₂)₃ und R², R³ = H.

Des weiteren kann unter Verwendung der obigen hochenergetischen Verbindungen der Erfindung auch ein carbocyclisches Gerüst, wie zum Beispiel Tetrahedrane, [3]-Prismane und Cubane, aufgebaut sein.

Eine hochenergetische Verbindung gemäß der Verbindung kann beispielsweise durch Umsetzen eines Mono- oder Bis-Trialkylsilylsubstituierten Alkins mit einem Halogentrinitromethan, durch Umsetzen eines Metallnitroformats mit einem Mono- oder Di-Halogenacetylen, oder durch Umsetzen einer Nitroniumverbindung mit einem Tris-oder Hexakis-Trialkylsilylsubstituierten Alkin hergestellt werden.

Die hochenergetische Verbindung der Erfindung eignet sich in besonders vorteilhafter Weise als Sprengmittel, Treibmittel, Oxidationsmittel und dergleichen.

Obige sowie weitere Merkmale und Vorteile der Erfindung werden aus der nachfolgenden Beschreibung eines bevorzugten, nicht-einschränkenden Beispiels einer erfindungsgemäßen Verbindung besser verständlich.

Als besonders geeignete und praktikable Verbindungen im Sinne der Erfindung haben sich herausgestellt:
a)- Trinitromethylacetylenderivate der Formel C₃R(NO₂)₃,
   wobei R ausgewählt ist aus der Gruppe bestehend aus H, NO₂, NO, NH₂, N(NO₂)₂, C(NO₂)₃ und einem Alkyl,
b)- Trinitromethylbutadiinderivate der Formel C₅R¹R²R³(NO₂)₃,
wobei R¹, R² und R³ jeweils ausgewählt sind aus der Gruppe bestehend aus H, NO₂, NO, NH₂, N(NO₂)₂, C(NO₂)₃ und einem Alkyl.

Zu a) wird insbesondere das Bis(trinitromethyl)acetylen (nachfolgend auch kurz als BTNMA bezeichnet) C₄(NO₂)₆ für R = C(NO₂)₃ vorgeschlagen, und zu b) wird insbesondere das Bis(trinitromethyl)butadün C₆H₂(NO₂)₆ für R¹ = C(NO₂)₃ und R², R³ = H vorgeschlagen.

Semiempirische Rechnungen haben für BTNMA eine im Vergleich zu HNE höhere Stabilität gezeigt. So sind die C-N - Bindungslängen in HNE im Durchschnitt um 2 pm länger als bei BTNMA (164,4 pm gegenüber 162,3 pm). Auch zeigt eine Darstellung der elektrostatischen Potentiale im Fall von BTNMA eine geringere positive Ladung über dem Molekül an, was nach Politzer ebenfalls eine höhere Stabilität und damit geringere Empfindlichkeit gegenüber Schlag und Reibung indiziert.

Ungeachtet dessen ist die Bildungsenthalpie von BTNMA aufgrund der energiereichen acetylenischen C2-Einheit um den Faktor 1,7 höher als bei HNE. Ein weiterer Vorteil von BTNMA gegenüber den anderen bislang bekannten Nitrokohlenstoffen, vor allem auch gegenüber dem thermisch sehr empfindlichen Tetranitroethylen, ist die generell hohe Reaktivität von Dreifachbindungen, die beim Aufbau von Kohlenstoffgerüsten genutzt werden kann.

Insofern eignen sich die oben genannten Verbindungen auch als Startmoleküle für zum Beispiel carbocyclische Gerüste, wie beispielsweise Tetrakis(trinitromethyl) tetrahedran (C(NO₂)₃)₄, Hexakis(trinitromethyl)-[3]-prisman (C(NO₂)₃)₆ oder Octakis (trinitromethyl)cuban (C(NO₂)₃)₈.

Zur Herstellung von BTNMA sind die folgenden Synthesewege denkbar.
a) Umsetzung von Bis(trimethylsilyl)acetylen mit einem Halogentrinitromethan gemäß z.B.
   (H₃C)₃Si-CC-Si(CH₃)₃ + 2 CIC(NO₂)₃ = 2 TMS-Cl + BTNMA
b) Umsetzung eines Metallnitroformats MC(NO₂)₃ mit in-situ präpariertem Dihalogenacetylen gemäß z.B.
   2 MC(NO₂)₃ + {C₂Cl₂} = 2 MCI + BTNMA
c) Umsetzung eines Hexakis(trimethylsilyl)but-2-in mit einer Nitroniumverbindung gemäß z.B.
   (TMS)₃C-C≡C-C(TMS)₃ + 6 NO₂X = 6 TMS-X + BTNMA
   wobei X = F, Cl, Br, I, BF₄ bzw. eine beliebige Lewissäure darstellt.

Analoge Umsetzungen führen auch zu allen anderen Verbindungen gemäß der vorliegenden Erfindung.

## Patentansprüche

1. Hochenergetische Verbindung, umfassend eine Alkineinheit,
bei welcher wenigstens ein Wasserstoffatom durch eine Trinitromethylgruppe substituiert ist.

2. Verbindung nach Anspruch 1,
bei welcher wenigstens ein weiteres Wasserstoffatom der Alkineinheit durch einen Rest substituiert ist, der ausgewählt ist aus der Gruppe bestehend aus NO₂, NO, NH₂, N(NO₂)₂ und einem Alkyl.

3. Verbindung nach Anspruch 1,
wobei die Verbindung ein Trinitromethylacetylenderivat der Formel C₃R(NO₂)₃ ist und R ausgewählt ist aus der Gruppe bestehend aus H, NO₂, NO, NH₂, N(NO₂)₂, C(NO₂)₃ und einem Alkyl.

4. Verbindung nach Anspruch 1,
wobei die Verbindung ein Trinitromethylbutadünderivat der Formel C₅R¹R²R³(NO₂)₆ ist und R¹, R² und R³ jeweils ausgewählt sind aus der Gruppe bestehend aus H, NO₂, NO, NH₂, N(NO₂)₂, C(NO₂)₃ und einem Alkyl.

5. Hochenergetische Verbindung, umfassend ein carbocyclisches Gerüst, das unter Verwendung einer Verbindung nach einem der Ansprüche 1 bis 4 aufgebaut ist.

6. Verfahren zur Herstellung einer hochenergetischen Verbindung nach einem der Ansprüche 1 bis 5, mit dem Verfahrensschritt des Umsetzens eines Mono- oder Bis-Trialkylsilylsubstituierten Alkins mit einem Halogentrinitromethan.

7. Verfahren zur Herstellung einer hochenergetischen Verbindung nach einem der Ansprüche 1 bis 5, mit dem Verfahrensschritt des Umsetzens eines Metallnitroformats mit einem Mono- oder Di-Halogenacetylen.

8. Verfahren zur Herstellung einer hochenergetischen Verbindung nach einem der Ansprüche 1 bis 5, mit dem Verfahrensschritt des Umsetzens einer Nitroniumverbindung mit einem Tris- oder Hexakis-Trialkylsilylsubstituierten Alkin.

9. Verwendung einer hochenergetischen Verbindung nach einem der Ansprüche 1 bis 5 zur Herstellung eines Sprengmittels, Treibmittels oder Oxidationsmittels.
